# EUROPEAN PATENT SPECIFICATION

(11) **EP 2 225 193 B1**
(45) Date of publication and mention of the grant of the patent: **26.02.2014**
(21) Application number: 08865312.6
(22) Date of filing: 18.12.2008
(51) Int. Cl.: C07C 403/08, C07C 403/24, C07C 29/56

(54) **PHOTOCHEMICAL ISOMERIZATION OF A PENT-2-EN-4-YN-1-OL**
PHOTOISOMERIZIERUNG EINES PENT-2-EN-4YN-1-OLS
ISOMÉRISATION PHOTOCHIMIQUE D'UN PENT-2-ÉN-4-YN-1-OL

(30) Priority: 20.12.2007 EP 07024755
(43) Date of publication of application: 08.09.2010
(73) Proprietor: DSM IP Assets B.V., 6411 TE Heerlen (NL)
(72) Inventor: BONRATH, Werner, 79115 Freiburg (DE); ONDRUSCHKA, Bernd, 04109 Leipzig (DE)
(74) Representative: Steck, Melanie
(86) International application number: PCT/EP2008/010811
(87) International publication number: WO 2009/080280

(56) References cited:
- EP-A- 1 167 331

## Description

The present invention relates to a process for photochemically isomerizing an E-pent-2-en-4-yn-1-ol to a Z-pent-2-en-4-yn-1-ol and vice versa.

Pent-2-en-4-yn-1-ol exists in two isomeric forms, the E-isomer and the Z-isomer. Both isomers of pent-2-en-4-yn-1-ols are important intermediates in industrial organic chemistry. A pent-2-en-4-yn-1-ol of particular interest is 3-methylpent-2-en-4-yn-1-ol; its Z-isomer is a useful intermediate, e.g. for the manufacture of Vitamin A, and its E-isomer is also a useful intermediate, e.g. for the manufacture of astaxanthin, zeaxanthin and further carotenoids. 3-Methylpent-2-en-4-yn-1-ol is obtained as an isomeric mixture of about 85 % of Z-3-methylpent-2-en-4-yn-1-ol and about 15 % of E-3-methylpent-2-en-4-yn-1-ol from the allylic rearrangement of 3-methylpent-1-en-4-yn-3-ol. The isomers may be separated from each other by physical means, e.g. by fractional distillation. Depending on the type of isomer desired there is a need for a process of converting the Z-isomer to the E-isomer (Z/E-isomerization) and vice versa.

EP-A 1 167 331 describes the catalytic isomerization of Z-3-methylpent-2-en-4-yn-l-ol to E-3-methylpent-2-en-4-yn-1-ol by bromine radicals.

It is the object of the present invention to provide an alternative process for isomerizing an E-pent-2-en-4-yn-1-ol to a Z-pent-2-en-4-yn-1-ol and vice versa. The alternative process should avoid the handling of halogen-containing materials.

The object is met by a process for photochemically isomerizing an E-pent-2-en-4-yn-1-ol according to formula (1) 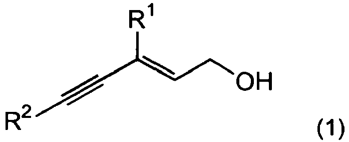 to a Z-pent-2-en-4-yn-1-ol according to formula (2) 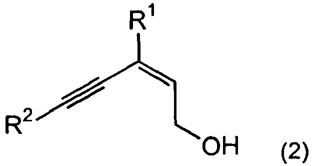 or photochemically isomerizing a Z-pent-2-en-4-yn-1-ol (2) to an E-pent-2-en-4-yn-1-ol (1) wherein
R¹ is selected from alkyl, aryl, and alkylaryl radicals,
R² is selected from H, alkyl, and aryl radicals;
comprising irradiating the E- or Z-isomer of the pent-2-en-4-yn-1-ol with UV light in the presence of a photosensitizer.

The formulae throughout the present application are represented by conventional line representation.

According to a preferred embodiment, R¹ in formulae (1) and (2) is selected from C₁ to C₁₅ alkyl radicals, preferably C₁ to C₅ alkyl radicals, and a phenyl radical; R² is selected from H, C₁ to C₁₅ alkyl radicals, preferably C₁ to C₅ alkyl radicals, and a phenyl radical.

According to the most preferred embodiment, R¹ is a methyl radical and R² is H. In this case, the Z- and E-isomers of the pent-2-en-4-yn-1-ol are Z-3-methylpent-2-en-4-yn-1-ol according to formula (3) and E-3-methylpent-2-en-4-yn-l-ol according to formula (4).

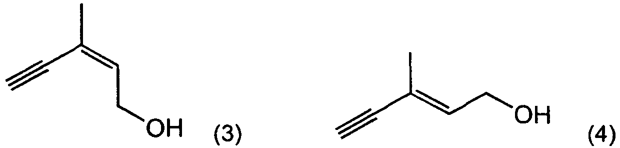

The type of UV light emitting source used to irradiate the E- or Z-isomer in the process of the present invention is not critical. Typically, the emitted UV light has at least main emission bands in the range of from 180 to 380 nm, preferably of from 220 to 280 nm, and more preferably of from 250 to 260 nm. One type of UV light source especially useful in the process of the present invention is a microwave-driven electrodeless UV lamp (microwave-driven electrodeless gas discharge lamp), more particularly a microwave-driven electrodeless mercury lamp, and preferably a microwave-driven electrodeless low pressure mercury lamp. Microwave-driven electrodeless lamps are generally described in "Microwaves in Organic Synthesis", Ed.: A. Loupy, Wiley-VCH, Weinheim 2006, Chapter 14.2. According to the emission spectrum of mercury the microwave-driven electrode less mercury lamps have main emission bands at about 254 nm and optionally about 185 nm, depending on the type of quartz glass enclosing the mercury vapor. In the presence of oxygen, emissions in the range of from 180 to 220 nm may lead to the formation of ozone which, however, does not have a negative effect on the isomerization. Microwave-driven electrodeless low pressure mercury lamps for use in the present invention are for example available from uv-technik, WOmbach, Germany.

In one embodiment of the present invention the E- or Z-isomer of the pent-2-en-4-yn-1-ol is under the at least temporary influence of a microwave radiation source during irradiation with UV light. One non-limiting possibility to realize the simultaneous UV and microwave irradiation of the isomer is choosing a set-up of the microwave-driven electrodeless UV lamp wherein the microwave radiation source is located to irradiate both the gas contained in the lamp cavity and generating the UV light by gas discharge and the isomers to be isomerized. However, it is not well understood what effects, if any, the microwave radiation has on the isomerization reaction.

As the microwave irradiation of the isomers is not essential for the present invention the set-up of the microwave-driven electrodeless UV lamp can also be chosen to only bring the gas contained in the lamp cavity under the influence of the microwave radiation to effect the gas discharge.

The output power of the microwave radiation source is not essential; for example it may be within the range of from 50 to 1200 W, preferably from 60 to 250 W.

The irradiation according to the present invention is performed in the presence of a photosensitizer. Preferably, the photosensitizer is selected from ketones such as benzophenone and Michler's ketone, and iodine. More preferably, the photosensitizer is selected from benzophenone and iodine.

The molar ratio of photosensitizer to pent-2-en-4-yn-1-ol is preferably at least 2:100 and more preferably at least 3:100. For practical and economical reasons the molar ratio of photosensitizer to pent-2-en-4-yn-1-ol is preferably less than 1:1, more preferably less than 70:100, and most preferably less than 50:100.

In one preferred embodiment of the present invention the irradiation is performed in the presence of an organic solvent. More preferably, the organic solvent is a polar organic solvent. Most preferably, the organic solvent is selected from alcohols, nitriles and mixtures thereof. The alcohols may by linear, branched or cyclic and preferably comprise 1 to 10 carbon atoms, more preferably 1 to 6 carbon atoms. The nitriles may be aliphatic or aromatic and preferably comprise 1 to 7 carbon atoms. The most preferred organic solvent is methanol and/or acetonitrile. Typically, both the pent-2-en-4-yn-1-ol and the photosensitizer are dissolved in the organic solvent.

Exemplary amounts of pent-2-en-4-yn-1-ol in the irradiated solution are within the range of from 0.1 to 50 weight %, preferably from 1 to 40 weight %, more preferably from 1 to 20 weight %, and most preferably from 1 to 10 weight %, based on the total weight of the solution. Exemplary concentrations of pent-2-en-4-yn-1-ol in the organic solvent are within the range of from 0.05 to 0.3 mol/l (M), preferably 0.1 to 0.2 mol/l (M).

The irradiation according to the present process can be performed at atmospheric, subatmospheric and superatmospheric pressures. Preferably, it is performed at pressures within the range of from atmospheric pressure up to 3 MPa, more preferably up to 2 MPa.

The irradiation can be performed under ambient atmosphere (air). The use of an inert gas is not required although inert gas conditions may be employed in some instances.

The irradiation temperature is not critical for the present invention; typically the irradiation is performed at a temperature within the range of from 0°C up to the boiling point of the used solvent at the pressure conditions. At atmospheric pressure, the irradiation is preferably performed at a temperature within the range of from 0°C to 80°C, preferably 20°C to 80°C. It is understood that the irradiation temperature should be selected to avoid or minimize any deterioration of the pent-2-en-4-yn-1-ol.

Exemplary irradiation times are within the range of from 5 to 120 min, preferably from 10 to 80 min, and most preferably from 15 to 60 min.

The reactor type used to irradiate the isomers of the pent-2-en-4-yn-1-ol is not critical for the present invention. Any type of reactor suitable for the described photoreaction can be used. The process can be preformed as continuous, semi-continuous or batch process.

Neither the E/Z-isomerization of an E-pent-2-en-4-yn-1-ol to a Z-pent-2-en-4-yn-1-ol nor the Z/E-isomerization in the reverse direction achieved by the irradiation according to the present process is complete. It is rather believed that the limiting ratio of E-isomer to Z-isomer that can be obtained theoretically by irradiating for a sufficient long time is determined by the thermodynamic equilibrium of the E- and Z-isomer at ambient conditions. For 3-methylpent-2-en-4-yn-1-ol, the equilibrium ratio of E- to Z-isomer is about 15:85 at 56°C. In practice, the equilibrium ratio is not necessarily reached by the irradiation according to the present invention.

The starting isomer(s) for the present process can be the E-isomer, the Z-isomer or a mixture of the E- and Z-isomers which does not correspond to the thermodynamic E/Z ratio. Starting with a neat isomer the theoretical obtainable maximal amount of the other isomer (often not reached in practice) is the amount reflecting the equilibrium ratio at ambient conditions. Starting with an isomeric mixture the irradiation shifts the E/Z ratio in direction of the equilibrium ratio.

If it is desired to obtain a neat isomer the desired isomerized isomer may be separated after the irradiation, preferably by physical means such as distillation, more preferably by fractional distillation. The not-isomerized isomer may then be irradiated again as described above and subsequently, the desired isomerized isomer may be separated. The irradiation and separation steps may be repeated as many times as desired and the separated isomerized isomer may be collected.

Z-3-methylpent-2-en-4-yn-1-ol obtained by a preferred embodiment of the present isomerization reaction is an intermediate product for the syntheses of vitamin A or vitamin A derivatives. It can be converted to vitamin A or a vitamin A derivative by various process steps well known to a person skilled in the art. One of the most economically successful processes for the preparation of vitamin A and its derivatives is the Isler synthesis of 1948. The conversion of Z-3-methylpent-2-en-4-yn-1-ol to vitamin A or a vitamin A derivative is for example described in US 2,451,739 to Otto Isler. Z-3-methylpent-2-en-4-yn-1-ol is coupled with the C₁₄ component 2-methyl-4-(2',6',6'-trimethyl-cyclohexen-1'-yl)-2-methyl-2-buten-1-al via a Grignard reaction to result in 1-hydroxy-3,7-dimethyl-6-hydroxy-9-(2',6',6'-trimethyl-cyclohexen-1'-yl-nonadiene-(2,7)-yne (4) (oxenyne). The oxenyne is first subjected to partial hydrogenation at the triple bond, preferably Lindlar hydrogenation; then esterification at the terminal hydroxyl group, preferably acetylation with acetic anhydride; followed by dehydration and allylic rearrangement. The resulting crude vitamin A ester, preferably vitamin A acetate, is then purified, preferably by crystallization. Thereafter, the vitamin A ester may be further reacted to obtain the desired vitamin A derivative, e.g. it may be hydrolyzed to obtain vitamin A.

E-3-methylpent-2-en-4-yn-1-ol obtained by another preferred embodiment of the present isomerization reaction also is a useful intermediate. It may be used to synthesize astaxanthin, zeaxanthin and further carotenoids. The conversion from E-3-methylpent-2-en-4-yn-1-ol to the desired carotenoid is carried out in various process steps as it is well known to the person skilled in the art. For example, E-3-methylpent-2-en-4-yn-1-ol is the C₆ starting component in the synthesis of astaxanthin wherein E-3-methylpent-2-en-4-yn-1-ol which is optionally protected is first reacted with a C₉ component, two equivalents of the resulting C₁₅ components are then reacted with a C₁₀ component to form astaxanthin (C₄₀). An illustrative synthesis of astaxanthin is described in EP-A 0 005 748. A different synthesis of astaxanthin also using E-3-methylpent-2-en-4-yn-1-ol as starting C₆ component is taught in CN-A-166 803. E-3-methylpent-2-en-4-yn-1-ol may also be used as the C₆ starting component in the synthesis of zeaxanthin: An exemplary technical preparation of zeaxanthin according to a 2(C₉ + C₆) + C₁₀ = C₄₀ construction wherein the E-3-methylpent-2-en-4-yn-1-ol is employed in its IPM-protected (isopropenyl methyl ether protected) form is described by E. Widmer et al. in Helv. Chim. Acta 1990, 73, 861.

The invention will now be further illustrated in the following non-limiting examples.

### EXAMPLES

### Starting materials:

E-3-methylpent-2-en-4-yn-1-ol, purity > 96 %, contained minor amounts of the Z-isomer
Z-3-methylpent-2-en-4-yn-1-ol, purity > 97 %, contained minor amounts of the E-isomer
Acetonitrile, benzophenone, Michler's ketone and iodine were obtained from Sigma-Aldrich GmbH (Fluka), Buchs, Switzerland, and used without any further purification.

### Analysis

The product mixtures are analyzed by gas chromatography (GC) using gas chromatograph HP-5890 from Hewlett Packard. GC-MS couplings together with the relevant test substances are used for the qualitative assignment of the reaction products. All GC evaluations are based on the principle of the inner GC standard (hexadecane). GC conditions: HP5 column: 30 m, 0.32 mm ID, 0.32 µm film thickness; injector: 200°C; detector: 280°C; oven: 70°C - 2 min - 10°C/min - 250°C; split: 80 ml; column prepressure: 5 psi; GC sample: 1 ml at 1 µl C₁₆.

### Examples 1 to 6

The E- or Z-isomer of 3-methylpent-2-en-4-yn-1-ol is dissolved in acetonitrile to result in a concentration of 0.1 mol/l. Different types and amounts of photosensitizers as indicated in Table 1 (B = benzophenone; M = Michler's ketone) are dissolved in 40 ml of the acetonitrile solution of the 3-methylpent-2-en-4-yn-1-ol. The solution obtained is added to a 100 ml glass flask that contains one electrodeless quartz lamp (from uv-technik, Wümbach, Germany; length about 45 to 50 cm, diameter 10 mm; filling: 0.3 µl Hg, 10 mbar Ar/Ne (75/25); main emission bands at 254 nm and 185 nm) and a magnetic stirring bar. The flask is further equipped with a reflux condenser. The flask is placed in the central cavity of a Discover® microwave generating apparatus from CEM Corporation, Matthews, North Carolina, U.S.A. Its power output is adjusted to 80 W (Example 1) or 130 W (Examples 2 to 6). The solution is irradiated under stirring and refluxing of the solvent for the irradiation times indicated in Table 1. The ratios of the E- and Z-isomer of 3-methylpent-2-en-4-yn-1-ol (E/Z ratio) in the starting mixtures (t = 0) and the mixtures obtained after irradiation are determined by gas chromatography (GC) as described above and the results are set forth in Table 1.

**Table 1**

| Ex. # | photosensitizer / amount | E/Z ratio at irradiation time t | | | | | | | |
|---|---|---|---|---|---|---|---|---|---|
| | | 0 | 20 min | 40 min | 60 min | 90 min | 2 h | 3 h | 4 h |
| 1 | B / 200 mg | 99.5:0.5 | 99.1:0.9 | 93:7 | 83:17 | 73:27 | 70:30 | nd | nd |
| 2 | B / 200 mg | 98.5:1.5 | 85:15 | 82:18 | 79:21 | 76:24 | 73:27 | 67:33 | 64:36 |
| 3 | B / 200 mg | 0.5:99.5 | 8:92 | 14:86 | 18:82 | nd | nd | nd | nd |
| 4 | B / 300 mg | 98.6:1.4 | 99.4:0.6 | 98:2 | 90:10 | nd | 60:40 | 40:60 | 40:60 |
| 5 | B / 25 mg | 85:15 | 80:20 | 81:19 | 76:33 | nd | 61:39 | nd | nd |
| 6 | M / 21 mg | 99.0:1.0 | 94:6 | 88:12 | 84:16 | nd | 66:34 | nd | nd |

| | | | | | | | | | |
|---|---|---|---|---|---|---|---|---|---|
| B = benzophenone; M = Michler's ketone; nd = not determined Ex. # = Example; min = minute(s); h = hour(s) | | | | | | | | | |

### Examples 7 to 10

The E-isomer of 3-methylpent-2-en-4-yn-1-ol is dissolved in acetonitrile to result in a concentration of 0.1 mol/l. Iodine as photosensitizer is dissolved in 40 ml of the acetonitrile solution of the 3-methylpent-2-en-4-yn-1-ol in varying amounts as indicated in Table 2. The solution obtained is added to a cylindrical quartz reactor (18 cm x 4 cm) that contains one electrodeless quartz lamp (from uv-technik, Wümbach, Germany; length about 45 to 50 cm, diameter 10 mm; filling: 0.3 µl Hg, 10 mbar Ar/Ne (75/25); main emission bands at 254 nm and 185 nm) and a magnetic stirring bar. The reactor is put into a protective container and sealed with a cap. The sealed container is then placed into a "Synthos 3000" microwave generating apparatus from Anton Paar GmbH, Graz, Austria (power output 1000 W). The solution is irradiated under stirring und heating for a total of 60 min according to the following scheme: heating up to 200°C within 7 min and maintaining at 200°C for further 54 min. The pressure within the sealed container at 200°C is about 2 MPa (20 bar). The ratios of the E- and Z-isomer of 3-methylpent-2-en-4-yn-1-ol (E/Z ratio) in the starting mixtures (t = 0) and the mixtures obtained after irradiation are determined by gas chromatography (GC) as described above and the results are set forth in Table 2.

**Table 2**

| Example | amount of I₂ | E/Z ratio at irradiation time t | |
|---|---|---|---|
| | | 0 | 60 min |
| 7 | 25 mg | 98.85:1.15 | 78:22 |
| 8 | 50 mg | 98.85:1.15 | 70:30 |
| 9 | 100 mg | 98.85:1.15 | 55:45 |
| 10 | 100 mg | 98.85:1.15 | 33:67 |

### Examples 11 to 13

A mixture of the E- and Z-isomer of 3-methylpent-2-en-4-yn-1-ol is dissolved in acetonitrile to result in a concentration of 0.1 mol/l. Benzophenone as photosensitizer is dissolved in 250 ml of the acetonitrile solution of the 3-methylpent-2-en-4-yn-1-ol in varying amounts as indicated in Table 3. The solution obtained is added into the jacket-type reaction chamber of an elongated standard UV photoreactor. The reaction chamber is surrounding an axially extending cavity wherein a 150 W mercury medium-pressure lamp is located. The solution is irradiated for 60 min and the temperature within the reactor is maintained at 25°C by cooling means controlled by a thermostat. The ratios of the E- and Z-isomer of 3-methylpent-2-en-4-yn-1-ol (E/Z ratio) in the starting mixtures (t = 0) and the mixtures obtained after irradiation are determined by gas chromatography (GC) as described above and the results are set forth in Table 3.

**Table 3**

| Example | amount of benzophenone | E/Z ratio at irradiation time t | |
|---|---|---|---|
| | | 0 | 60 min |
| 11 | 50 mg | 90:10 | 80:20 |
| 12 | 100 mg | 90:10 | 70:30 |
| 13 | 300 mg | 90:10 | 70:30 |

## Claims

1. A process for photochemically isomerizing an E-pent-2-en-4-yn-1-ol according to formula (1) 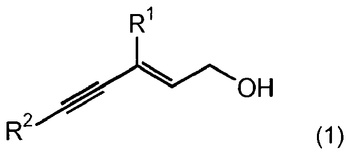 to a Z-pent-2-en-4-yn-1-ol according to formula (2) 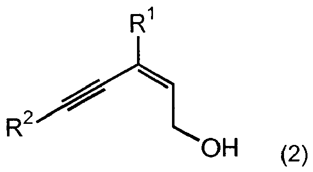 or photochemically isomerizing a Z-pent-2-en-4-yn-1-ol (2) to an E-pent-2-en-4-yn-1-ol (1) wherein
R¹ is selected from alkyl, aryl, and alkylaryl radicals,
R² is selected from H, alkyl, and aryl radicals;
comprising irradiating the E- or Z-isomer of the pent-2-en-4-yn-l-ol with UV light in the presence of a photosensitizer.

2. The process according to claim 1, wherein the E- or Z-isomer of the pent-2-en-4-yn-1-ol is irradiated with a microwave driven electrodeless UV lamp.

3. The process according to claim 1 or 2, wherein the E- or Z-isomer of the pent-2-en-4-yn-1-ol is under the at least temporary influence of a microwave radiation source during irradiation with UV light.

4. The process according to any of the preceding claims, wherein the microwave-driven electrodeless UV lamp is a microwave-driven electrodeless mercury lamp, preferably wherein the microwave-driven electrodeless UV lamp is a low pressure mercury lamp.

5. The process according to any of the preceding claims, wherein the photosensitizer is selected from ketones and iodine, preferably wherein the photosensitizer is benzophenone.

6. The process according to any of the preceding claims, wherein the irradiation is performed in the presence of an organic solvent.

7. The process according to claim 6, wherein the organic solvent is a polar organic solvent, preferably wherein the organic solvent is selected from alcohols, nitriles, and mixtures thereof, more preferably wherein the organic solvent is acetonitrile or methanol.

8. The process according to any of the preceding claims comprising irradiating a mixture comprising both the Z-pent-2-en-4-yn-1-ol (1) and E-pent-2-en-4-yn-1-ol (2).

9. The process according to any of the preceding claims, wherein R¹ is a methyl radical and R² is H.

10. The process according to claim 9, wherein E-3-methylpent-2-en-4-yn-1-ol is isomerized to Z-3-methylpent-2-en-4-yn-1-ol.

11. The process according to claim 9, wherein to Z-3-methylpent-2-en-4-yn-1-ol is isomerized to E-3-methylpent-2-en-4-yn-1-ol.

12. The process according to any of the preceding claims further comprising subsequent separation of the isomerized isomer, preferably further comprising subsequent separation of the isomerized isomer by fractional distillation.

13. A process comprising the multiple subsequent performance of the process according to claim 12 and collection of the separated isomerized isomer.

14. A process of preparing vitamin A or a vitamin A derivative, which process comprises photochemically isomerizing E-3-methylpent-2-en-4-yn-1-ol to Z-3-methylpent-2-en-4-yn-1-ol according to claim 10, isolating the Z-3-methylpent-2-en-4-yn-1-ol and converting it to vitamin A or a vitamin A derivative.

15. A process of preparing a carotenoid, which process comprises photochemically isomerizing Z-3-methylpent-2-en-4-yn-1-ol to E-3-methylpent-2-en-4-yn-1-ol according to claim 11, isolating the E-3-methylpent-2-en-4-yn-1-ol and converting it to the carotenoid.

## Patentansprüche

1. Verfahren zur photochemischen Isomerisierung eines E-Pent-2-en-4-in-1-ols gemäß Formel (1) 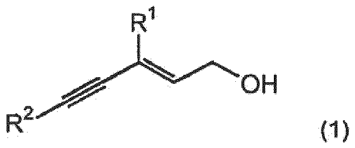 zu einem Z-Pent-2-en-4-in-1-ol gemäß Formel (2) 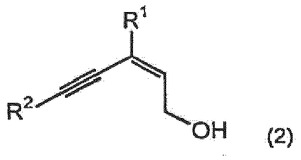 oder zur photochemischen Isomerisierung eines Z-Pent-2-en-4-in-1-ols gemäß Formel (2) zu einem E-Pent-2-en-4-in-1-ols gemäß Formel (1), wobei
R¹ aus Alkyl-, Aryl- und Alkylarylresten ausgewählt ist,
R² aus H, Alkyl- und Arylresten ausgewählt ist; bei dem man das E- bzw. Z-Isomer des Pent-2-en-4-in-1-ols in Gegenwart eines Photosensibilisators mit UV-Licht bestrahlt.

2. Verfahren nach Anspruch 1, bei dem man das E- bzw. Z-Isomer des Pent-2-en-4-in-1-ols mit einer mikrowellengetriebenen elektrodenlosen UV-Lampe bestrahlt.

3. Verfahren nach Anspruch 1 oder 2, bei dem das E-bzw. Z-Isomer des Pent-2-en-4-in-1-ols während der Bestrahlung mit UV-Licht unter dem zumindest vorübergehenden Einfluss einer Mikrowellenstrahlungsquelle steht.

4. Verfahren nach einem der vorhergehenden Ansprüche, bei dem es sich bei der mikrowellengetriebenen elektrodenlosen UV-Lampe um eine mikrowellengetriebene elektrodenlose Quecksilberlampe und vorzugsweise um eine Niederdruck-Quecksilberlampe handelt.

5. Verfahren nach einem der vorhergehenden Ansprüche, bei dem man den Photosensibilisator aus Ketonen und Iod auswählt, wobei sich bei dem Photosensibilisator vorzugsweise um Benzophenon handelt.

6. Verfahren nach einem der vorhergehenden Ansprüche, bei dem man die Bestrahlung in Gegenwart eines organischen Lösungsmittels durchführt.

7. Verfahren nach Anspruch 6, bei dem es sich bei dem organischen Lösungsmittel um ein polares organisches Lösungsmittel handelt, wobei das organische Lösungsmittel vorzugsweise aus Alkoholen, Nitrilen und Mischungen davon ausgewählt wird und es sich bei dem organischen Lösungsmittel besonders bevorzugt um Acetonitril oder Methanol handelt.

8. Verfahren nach einem der vorhergehenden Ansprüche, bei dem man ein Gemisch, dass sowohl das Z-Pent-2-en-4-in-1-ol (1) als auch das E-Pent-2-en-4-in-1-ol (2) umfasst, bestrahlt.

9. Verfahren nach einem der vorhergehenden Ansprüche, bei dem R¹ für einen Methylrest steht und R² für H steht.

10. Verfahren nach Anspruch 9, bei dem man E-3-Methyl-pent-2-en-4-in-1-ol zu Z-3-Methylpent-2-en-4-in-1-ol isomerisiert.

11. Verfahren nach Anspruch 9, bei dem man Z-3-Methyl-pent-2-en-4-in-1-ol zu E-3-Methylpent-2-en-4-in-1-ol isomerisiert.

12. Verfahren nach einem der vorhergehenden Ansprüche, bei dem man ferner anschließend das isomerisierte Isomer abtrennt, vorzugsweise durch fraktionierte Destillation.

13. Verfahren, bei dem man das Verfahren gemäß Anspruch 12 mehrmals aufeinanderfolgend durchführt und das abgetrennte isomerisierte Isomer sammelt.

14. Verfahren zur Herstellung von Vitamin A oder einem Vitamin-A-Derivat, bei dem man E-3-Methylpent-2-en-4-in-1-ol gemäß Anspruch 10 photochemisch zu Z-3-Methylpent-2-en-4-in-1-ol isomerisiert, das Z-3-Methylpent-2-en-4-in-1-ol isoliert und es in Vitamin A oder ein Vitamin-A-Derivat umwandeln.

15. Verfahren zur Herstellung eines Carotinoids, bei dem man Z-3-Methylpent-2-en-4-in-1-ol gemäß Anspruch 11 photochemisch zu E-3-Methylpent-2-en-4-in-1-ol isomerisiert, das E-3-Methylpent-2-en-4-in-1-ol isoliert und es in das Carotinoid umwandelt.

## Revendications

1. Procédé pour l'isomérisation photochimique d'un E-pent-2-én-4-yn-1-ol selon la formule (1) 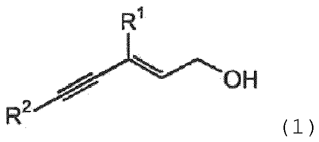 en Z-pent-2-én-4-yn-1-ol selon la formule (2) 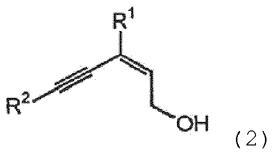 ou l'isomérisation photochimique d'un Z-pent-2-én-4-yn-1-ol (2) en E-pent-2-én-4-yn-1-ol (1) où
R¹ est choisi parmi des radicaux alkyle, aryle, et alkylaryle,
R² est choisi parmi des radicaux H, alkyle, et aryle ; comprenant l'irradiation de l'isomère E ou Z du pent-2-én-4-yn-1-ol avec de la lumière UV en présence d'un photosensibilisateur.

2. Procédé selon la revendication 1, dans lequel l'isomère E ou Z du pent-2-én-4-yn-1-ol est irradié avec une lampe UV sans électrode excitée par micro-onde.

3. Procédé selon la revendication 1 ou 2, dans lequel l'isomère E ou Z du pent-2-én-4-yn-1-ol est sous l'influence au moins temporaire d'une source de rayonnement micro-onde pendant l'irradiation avec de la lumière UV.

4. Procédé selon l'une quelconque des revendications précédentes, dans lequel la lampe UV sans électrode excitée par micro-ondes est une lampe à mercure sans électrode excitée par micro-ondes, de préférence dans lequel la lampe UV sans électrode excitée par micro-ondes est une lampe à mercure basse pression.

5. Procédé selon l'une quelconque des revendications précédentes, dans lequel le photosensibilisateur est choisi parmi des cétones et l'iode, de préférence dans lequel le photosensibilisateur est la benzophénone.

6. Procédé selon l'une quelconque des revendications précédentes, dans lequel l'irradiation est effectuée en présence d'un solvant organique.

7. Procédé selon la revendication 6, dans lequel le solvant organique est un solvant organique polaire, de préférence dans lequel le solvant organique est choisi parmi des alcools, des nitriles, et des mélanges de ceux-ci, plus préférablement dans lequel le solvant organique est l'acétonitrile ou le méthanol.

8. Procédé selon l'une quelconque des revendications précédentes comprenant l'irradiation d'un mélange comprenant à la fois le Z-pent-2-én-4-yn-1-ol (1) et le E-pent-2-én-4-yn-1-ol (2).

9. Procédé selon l'une quelconque des revendications précédentes, dans lequel R¹ est un radical méthyle et R² est H.

10. Procédé selon la revendication 9, dans lequel le E-3-méthylpent-2-én-4-yn-1-ol est isomérisé en Z-3-méthylpent-2-én-4-yn-1-ol.

11. Procédé selon la revendication 9 dans lequel le Z-3-méthylpent-2-én-4-yn-1-ol est isomérisé en E-3-méthylpent-2-én-4-yn-1-ol.

12. Procédé selon l'une quelconque des revendications précédentes comprenant en outre la séparation consécutive de l'isomère isomérisé, de préférence comprenant en outre la séparation consécutive de l'isomère isomérisé par distillation fractionnée.

13. Procédé comprenant la conduite consécutive multiple du procédé selon la revendication 12 et la collecte de l'isomère isomérisé séparé.

14. Procédé de préparation de la vitamine A ou d'un dérivé de vitamine A, ledit procédé comprenant l'isomérisation photochimique de E-3-méthylpent-2-én-4-yn-1-ol en Z-3-méthylpent-2-én-4-yn-1-ol selon la revendication 10, l'isolement du Z-3-méthylpent-2-én-4-yn-1-ol et sa conversion en vitamine A ou un dérivé de vitamine A.

15. Procédé de préparation d'un caroténoïde, ledit procédé comprenant l'isomérisation photochimique de Z-3-méthylpent-2-én-4-yn-1-ol en E-3-méthylpent-2-én-4-yn-1-ol selon la revendication 11, l'isolement du E-3-méthylpent-2-én-4-yn-1-ol et sa conversion en caroténoïde.
